# EUROPEAN PATENT APPLICATION

(11) **EP 1 923 058 A1**
(43) Date of publication of application: **21.05.2008**
(21) Application number: 06121299.9
(22) Date of filing: 26.09.2006
(51) Int. Cl.: A61K 9/32, A61K 9/36, A61K 31/135, A61P 37/06

(54) **Coated pharmaceutical composition comprising an S1P agonist or modulator**

(71) Applicant: Novartis AG, 4056 Basel (CH)
(72) Inventor: The designation of the inventor has not yet been filed
(74) Representative: Vögeli-Lange, Regina

(57) **Abstract**

The present invention provides an oral pharmaceutical composition comprising an S1P agonist and/or modulator having a coating.

## Description

The present invention relates to an oral composition comprising an S1P agonist and/or modulator.

In particular, the present invention relates to a solid or liquid composition comprising an S1P agonist having a coating.

The oral route is often the most convenient route for drug administration, but unfortunately many patients have difficulties in swallowing due to a unpleasant taste of the dosage form. This can be overcome by applying a unique coating to a tablet or pellet cores, or to the surface of a capsule, hence improving compliance by reducing or masking an unpleasant taste.

Accordingly, the present invention provides an oral pharmaceutical composition comprising an S1 P agonist and/or modulator having a coating.

The solid compositions mentioned herein may take the form of pellets of differening size, whereby the coating is applied to individual pellets, which may be present in a plurality, for example in a capsule or sachet.

The solid compositions of the present invention may be formed from powder ingredients, which may be micronised, and may be compressed into compositions of differing hardness.

In one embodiment, the powder constituents of the compressed composition are coated prior to compression.

In another embodiment, the compressed composition is coated after compression.

In another embodiment of the present invention the coating is applied both before and after compression.

Liquid oral compositions of the present invention comprise capsules containing the liquid composition, where the capsule comprise a coating.

In one embodiment, the coating is applied to the outer surface of the capsule.

In another embodiment, the coating is dispersed within the outer surface of the capsule.

Capsules are not however limited to liquid contents and may comprise solid compositions in the form of powders, pellets, heterogeneous suspensions in addition to homogeneous liquids.

In particular, the coating compositions of the present invention are for use on tablet compositions, referred to herein and exemplified as core tablets.

### Core tablet

In one embodiment of the present invention, the coating composition is used to coat a compressed core tablet comprising an S1 P agonist and/or modulator.

The core tablet may be any solid formulation for oral administration.

The term "core" comprises, in a wide sense, not only tablets, pellets or granules but also capsules, e.g. soft or hard capsules of gelatine or starch. Such cores may be produced in a conventional manner.

When tablet cores are used they have preferably a hardness of from ca. 10 to 70 N. The tablet core may tensile strength of less than 38N/cm², for example as low as 22N/cm².

In one aspect of the present invention, there is provided a means for increasing the hardness of a core tablet comprising an S1P agonist and/or modulator by applying a coating as described herein.

The coating may therefore provide a means for obtaining tablets comprising an S1 P agonist and/or modulator having good structural integrity which are formed from cores having a tensile strength of less than 38N/cm2 (2.5kP) i. e. cores that are so weak that previously they would have been regarded as too weak for practical use. The cores may have a tensile strength less than 30N/cm2 (2.0kP), preferably less than 22N/cm2 (1.5kP).

The cores may be formed by light compression and enable coated components and fragile components, such as capsules, to be used within the compression blend with little or no damage.

The tablet core comprises an adjuvant and an S1 P agonist and/or modulator.

The tablet core may comprise conventional tabletting ingredients, including diluents, disintegrants, lubricants, wetting agents, glidants, surfactants, release aids, colourants, gas producers, etc.

The core tablet may be formulated by any known formulation known to the skilled man.

The core tablet may be composed of, but not limited to, fillers such as, polyols, powdered mannitol, for example, or other saccharides or sugars, sugar alcohols etc, e.g. lactose, sucrose, dextrose, mannitol , starch.

The core tablet compositions may also include, or alternatively include, binders such as PVP e.g. cellulose, microcrystalline cellulose, polyethylene glycols, polyvinylpyrrolidone, starch mucilage, acacia, alginic acid, carboxymethylcellulose, hydroxyethylcellulose, hydroxypropylcellulose, dextrin, ethylcellulose, gelatin, glucose, guar gum, hydroxypropylmethylcellulose, magnesium aluminium silicate, kaltodectrin, methylcellulose, polyethylene oxide, povidone, sodium alginate and hydrogenated vegetable oils.

The core tablet compositions may also include, or alternatively include, disintegrants (with or without effervescent agents), e.g. cross-linked sodium carboxymethyl cellulose (Crosscarmellose), crosspovidone, sodium starch glycolate.

The core tablet compositions may also include, or alternatively include, lubricants e.g. magnesium stearate, calcium stearate, sodium stearyl fumarate, colloidal silica, talc.

In one embodiment, the core tablet comprises 1.5-2 % lubricant, e.g. magnesium stearate, calcium stearate.

The core tablet compositions may also include, or alternatively include, glidants, e.g. silica.

The core tablet compositions may also include, or alternatively include, surfactants, e.g. sodium lauryl sulphate, docusate sodium.

The core tablet compositions may also include, or alternatively include, flavoring agents.

The core tablet compositions may also include, or alternatively include, gas producers, e.g. sodium bicarbonate, citric acid.

The core tablet compositions may also include, or alternatively include, sweeteners.

The core tablet compositions may also include, or alternatively include, pH adjusting agents, e.g. citric acid, fumaric acid.

The tablet core may comprise a release rate controlling additive. For example, the drug may be held within a hydrophobic polymer matrix so that it is gradually leached out of the matrix upon contact with body fluids.

Alternatively, the drug may be held within a hydrophilic matrix which gradually or rapidly dissolves in the presence of body fluid. The tablet core may comprise two or more layers having different release properties. The layers may be hydrophilic, hydrophobic or a mixture of hydrophilic and hydrophobic layers. Adjacent layers in a multilayer tablet core may be separated by an insoluble barrier layer or hydrophilic separation layer. An insoluble barrier layer may be formed of materials used to form the insoluble casing. A hydrophilic separation layer may be formed from a material more soluble than the other layers of the tablet core so that as the separation layer dissolves the release layers of the tablet core are exposed.

Suitable release rate controlling polymers include polymethacrylates, ethylcellulose, hydroxypropylmethylcellulose, methylcellulose, hydroxyethylcellulose, hydroxypropylcellulose, sodium carboxymethylcellulose, calcium carboxymethylcellulose, acrylic acid polymer, polyethylene glycol, polyethylene oxide, carrageenan, cellulose acetate, zein etc.

The core tablet may additionally include materials which swell on contact with aqueous liquids, and which may be included in the composition, include polymer materials include from cross-linked sodium carboxymethylcellulose, cross-linked hydroxypropylcellulose, high molecular weighthydroxypropylcellulose, carboxymethylamide, potassium methacrylatedivinylbenzene copolymer, polymethylmethacrylate, cross-linked polyvinylpyrrolidone and high molecular weight polyvinylalcohols.

The core tablet pharmaceutical compositions of the present invention may be produced by standard processes, for instance by conventional mixing, granulating, dissolving or lyophilizing processes. Procedures which may be used are known in the art, e.g. those described in L. Lachman et al. The Theory and Practice of Industrial Pharmacy, 3rd Ed, 1986, H. Sucker et al, Pharmazeutische Technologie, Thieme, 1991, Hagers Handbuch der pharmazeutischen Praxis, 4th Ed. (Springer Verlag, 1971) and Remington's Pharmaceutical Sciences, 13th Ed., (Mack Publ., Co., 1970) or later editions.

In one embodiment of the present invention, there is provided a core composition comprising a microcrystalline cellulose and an S1 P receptor agonist, in the absence of a sugar alcohol.

The core tablet may comprise additional pharmaceutically active ingredients in addition to an S1 P agonist and/or modulator.

Where the solid composition is in the form of pellets or granules, these may, after application of the coating as described hereinafter, be used as such or to fill capsules, e.g. hard gelatine capsules or other storage means, for example sachets prior to administration.

Pellets and granules may be from 2mm to 0.3mm in diameter, for example, a "normal pellet" has a size of 1-0.6mm and a "bead pellet" has a size of 0.4 to 0.8mm.

### Tablet coating

A coating may be powder or liquid based.

The coating composition of the present invention comprises
(a) one or more polymer resins
(b) one or more metal oxides

A coating composition may have both suitable electrical properties and be fusible at a temperature suitable for use as the coating material in the coating of pharmaceutical tablet cores.

Examples of a polymer resin may include, but is not limited to, polymethacrylates, for example ammonio methacrylate, cellulose and its derivatives, cellulose ethers and esters and cellulose acetate phthalate.

Preferably, the polymer resin is non-conductive.

A coating composition of the present invention may comprise polyethylene glycol or a sugar alcohol, e.g. xylitol.

A coating composition may also include, or alternatively include, other possible materials include waxes and oils or alcohols of waxes or oils, poloxamers, alkyl phthalates, for example diethylphthalate, citric acid or esters.

A coating composition may also include, or alternatively include, one or more of acrylic acid, polymers and co-polymers of acrylic acid and their derivatives, for example polymethyl acrylate, polyalkenes and their derivatives, including esters and aryl-esters and their derivatives, polyvinyl alcohols and esters, cellulose and its derivatives, e.g. cellulose ethers and cellulose esters (either cross-linked or uncross-linked) for example ethyl cellulose, and one or more enteric polymers, e.g. cellulose acetate phthalate, hydroxypropyl methyl cellulose phthalate, hydroxypropylcellulose, one or more biodegradable polymers, e.g. one or more of polylactides, polyglycolides, polyhydroxybutyrates, polyhydroxyvalyrate, ethylene vinyl acetate copolymers, and polyanhydrides (homo or hetero polymers), polyethylene oxide.

A coating composition may also include, or alternatively include, a dispersing agent, e.g. sodium lauryl sulphate, docusate sodium, Tweens (sorbitan fatty acid esters), poloxamers and cetostearylalcohol.

A coating composition may also include, or alternatively include, an anti-friction component to reduce the frictional and/or other forces between the particles of the powder coating material to improve the flowability of the powder, e.g. titanium dioxide, colloidal silicon dioxide, talc or starch or a combination of those.

A coating composition may also include, or alternatively include, a disintegrator, e.g. sodium starch glycolate (cross-linked), sodium carboxymethylcellulose (cross-linked), native starch, cross-linked polyvinyl pyrrolidone (Crosprovidone), sodium carbonate, sodium hydrogen carbonate and sodium glycinate.

A coating composition may also include, or alternatively include, colourants, e.g. metal oxides or lakes (e.g. aluminium lakes), iron oxide, dyes.

A coating composition may also include, or alternatively include, taste modifiers, e.g. aspartame, acesulfame k, cyclamates, saccharin, sugars and sugar alcohols.

A coating composition may also include, or alternatively include, flavourings.

In one embodiment of the present invention, the coating comprises:
(a) a methacrylic acid co-polymer
(b) a cellulose
(c) one or more metal oxides

In one embodiment of the present invention, the coating improves the structural integrity consequently increasing the hardness of the tablets.

The coating mixture may be prepared by melt-extrusion of a mixture of polymer, coloring agent and other additives and than further micronization of the produced melt-extrudate is necessary (7-10 micron). The coating powders are stable in appropriate packaging and can be used to coat product for at least one year after manufacture.

The coating extending over the tablet core results from the electrostatic deposition of a powder comprising fusible particles.

This technique allows the formation of a thin, continuous film over surface areas of the tablet core. In general, the film will cover from 25 to 100% preferably 50 to 100% of the surface area of the tablet core. The resulting tablet preferably has a tensile strength of at least 50N/cm2, 60N/cm2 and most preferably at least 70N/cm2.

### The coating process:

First the core is fixed (vacuum) on a wheel, charged, transported through the coating chamber and the opposite charged coating powder is attached to the core surface. Then this powder layered core is transported on the wheel to an IR lamp were the coat melts. Then the core is transferred to the adjacent second wheel and the process is repeated for the bottom part of the tablet core.
Film thickness: 20-50um.

Typical coat weights are 3-4% of the core weight eg. 6mg coat on a 10mm bi-convex tablet. The max. coat weight for a 12mm round core is 20mg.The coat is preferably highly homogenous and preferably has a uniform thickness.

Heating step: This includes heating up the tablets from room temperature, so the temperature at the surface of the tablet peaks at approximately 100°C and in the tablet core approximately 70°C for about 20s. The total thermal exposure is much less that for conventional film coating(60-70°C for 1-2hours).

Preferably, the coating composition is non-conductive and has a melting point below 103°C, e.g. melts within 5 seconds at 130°C.

Preferably, the core is conductive. If it is not conductive, the core preferably contains 3-5% of a salt, for example NaCl, KCl, Lactilol, citric acid.

In one embodiment of the present invention, the S1P agonist and/or modulator provides conductive properties to the tablet core.

Therefore, there is provided a process of manufacturing a coated composition comprising an S1 P agonist and/or modulator, the process comprising the step of:
(a) making a composition comprising an S1 P agonist
(b) applying an electrostatic coating to the compositing
(c) fixing the coating.

In one particularly preferred process, the S1P agonist represents at least 50% of the conductive component of the core composition, for example at least 60%, typically more than 75%.

The S1 P agonist may be the only conductive component in the core composition.

The coating may be also applied by a spaying technique. Conveniently the cores may be treated at room temperature or warmed up to 40 °C e.g. by means of warm air of 40 ° up to 70 °C, before spraying. To avoid a sticking of the cores the spray procedure is preferably interrupted at certain time intervals and the cores then warmed up again. It is, however, also possible to proceed without interruption of the spray procedure, e.g. by automatic regulation of the spray amount taking into account the temperature of exhaust air and/or cores.

Various designs, prints, shapes etc may be applied to the coating to provide the final product with a distinctive look.

The spray pressure may vary within wide ranges, in general satisfactory results are obtained with a spray pressure of from about 1 to about 1.5 bar.

Preferably, the components of both the core tablet and the coating are micronised.

### S1P agaonists and/or modulators

Sphingosine-1 phosphate (hereinafter "S1P") is a natural serum lipid. Presently there are 8 known S1P receptors, namely S1 P1 to S1P8. S1P receptor agonists have accelerating lymphocyte homing properties.

S1P receptor agonists are immunomodulating compounds which elicit a lymphopenia resulting from a re-distribution, preferably reversible, of lymphocytes from circulation to secondary lymphatic tissue, evoking a generalized immunosuppression. Naive cells are sequestered, CD4 and CD8 T-cells and B-cells from the blood are stimulated to migrate into lymph nodes (LN) and Peyer's patches (PP), and thus infiltration of cells into transplanted organs is inhibited.

The various known S1 P receptor agonists show structural similarities, which result in related problems in providing a suitable formulation. In particular, there is a need for an S1P receptor agonist containing formulation which is well-adapted for oral administration in a solid form, e.g. as a tablet or capsule.

In one instance, there is a need for a S1 P receptor agonist containing oral formulation which can easily be swallowed, e.g. by children or older patients.

The compositions provide a convenient means of systemic administration of S1P receptor agonists, do not suffer from the disadvantages of liquid formulations for injection or oral use, and have good physicochemical and storage properties. In particular, the compositions of the present invention may show a high level of uniformity in the distribution of the S1P receptor agonist throughout the composition, as well as high stability. The compositions of the invention may be manufactured on high speed automated equipment, and thus do not require hand encapsulation.

S1P receptor agonists are typically sphingosine analogues, such as 2-substituted 2-aminopropane-1,3-diol or 2-amino-propanol derivatives. Examples of appropriate S1P receptor agonists are, for example:
Compounds as disclosed in EP627406A1, e.g. a compound of formula I 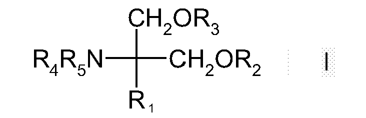 wherein
   R₁ is a straight- or branched (C₁₂₋₂₂) carbon chain,
   which may have in the chain a bond or a hetero atom selected from a double bond, a triple bond, O, S, NR₆, wherein R₆ is H, alkyl, aralkyl, acyl or alkoxycarbonyl, and carbonyl; and/or
   which may have as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxyimino, hydroxy or carboxy; or
   R₁ is a phenylalkyl wherein alkyl is a straight- or branched (C₆₋₂₀)carbon chain; or a phenylalkyl wherein alkyl is a straight- or branched (C₁₋₃₀)carbon chain wherein said phenylalkyl is substituted by
   a straight- or branched (C₆₋₂₀)carbon chain optionally substituted by halogen,
   a straight- or branched (C₆₋₂₀)alkoxy chain optionally substitued by halogen,
   a straight- or branched (C₆₋₂₀)alkenyloxy;
   phenylalkoxy, halophenylalkoxy, phenylalkoxyalkyl, phenoxyalkoxy or phenoxyalkyl;
   cycloalkylalkyl substituted by a straight- or branched (C₆₋₂₀)alkyl chain;
   heteroarylalkyl substituted by a straight- or branched (C₆₋₂₀)alkyl chain;
   heterocyclic alkyl wherein said alkyl is a straight- or branched (C₆₋₂₀)carbon chain;
   or
   heterocyclic alkyl substituted by a straight- or branched (C₂₋₂₀)alkyl chain,
   and wherein the alkyl moiety may have in the carbon chain, a bond or a heteroatom selected from a double bond, a triple bond, O, S, sulfinyl, sulfonyl, or NR₆, wherein R₆ is as defined above;
   and as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxy or carboxy, and
   each of R₂, R₃, R₄ and R₅, independently, is H, C₁₋₄ alkyl or acyl
   or a pharmaceutically acceptable salt thereof;
Compounds as disclosed in EP 1002792A, e.g. a compound of formula II 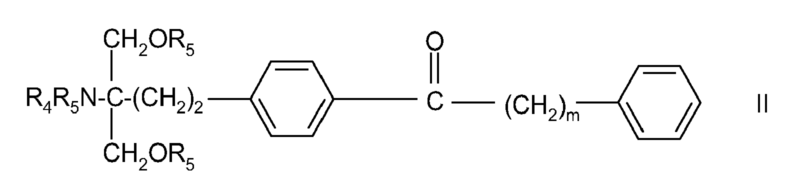 wherein
   m is 1 to 9; and
   each of R₂, R₃, R₄ and R₅, independently, is H, alkyl or acyl;
   or a pharmaceutically acceptable salt thereof;
Compounds as disclosed in EP0778263 A1, e.g. a compound of formula III 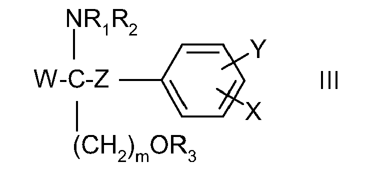 wherein
   W is H; straight chain or branched (C₁₋₆)alkyl, (C₂₋₆)alkenyl or (C₂₋₆)alkynyl; unsubstituted or by OH substituted phenyl; R₄O(CH₂)ₙ; or straight chain or branched (C₁₋₆)alkyl substituted by 1 to 3 substituents selected from the group consisting of halogen, cycloalkyl, phenyl or phenyl substituted by OH;
   X is H or unsubstituted or substituted straight chain alkyl having a number p of carbon atoms or unsubstituted or substituted straight chain alkoxy having a number (p-1) of carbon atoms, e.g. substituted by 1 to 3 substitutents selected from the group consisting of alkyl, OH, alkoxy, acyloxy, amino, alkylamino, acylamino, oxo, haloalkyl, halogen, unsubstituted phenyl or phenyl substituted by 1 to 3 substituents selected from the group consisting of alkyl, OH, alkoxy, acyl, acyloxy, amino, alkylamino, acylamino, haloalkyl and halogen;
   Y is H, alkyl, OH, alkoxy, acyl, acyloxy, amino, alkylamino, acylamino, haloalkyl or halogen, Z is a single bond or a straight chain alkylene having a number or carbon atoms of q,
   each of p and q, independently, is an integer of 1 to 20, with the proviso of 6≤p+q≤23, m is 1, 2 or 3,
   n is 2 or 3,
   each of R₁, R₂, R₃ and R₄, independently, is H, alkyl or acyl,
   or a pharmaceutically acceptable salt thereof.
Compounds as disclosed in WO02/18395, e.g. a compound of formula lVa or lVb 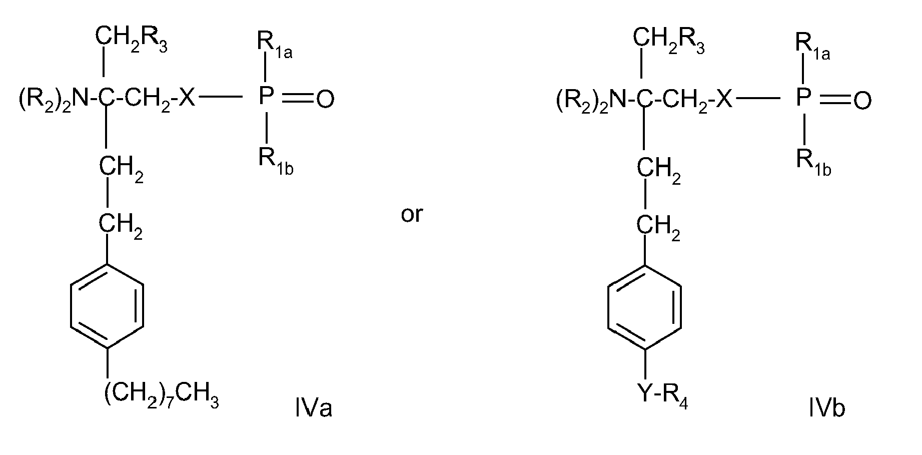 wherein
   X is O, S, NR₁ or a group -(CH₂)ₙ-, which group is unsubstituted or substituted by 1 to 4 halogen;
   n is 1 or 2,
   R₁ is H or (C₁₋₄)alkyl, which alkyl is unsubstituted or substituted by halogen;
   R₁ₐ is H, OH, (C₁₋₄)alkyl or O(C₁₋₄)alkyl wherein alkyl is unsubstituted or substituted by 1 to 3 halogen;
   R_{1b} is H, OH or (C₁₋₄)alkyl, wherein alkyl is unsubstituted or substituted by halogen;
   each R₂ is independently selected from H or (C₁₋₄)alkyl, which alkyl is unsubstituted or substitued by halogen;
   R₃ in case of a compound of formula IVa is H, OH, halogen or O(C₁₋₄)alkyl wherein alkyl is unsubstituted or substituted by halogen;
   R₃ in case of a compound of formula IVb is H, OH, halogen, (C₁₋₄)alkyl wherein alkyl is unsubstituted or substituted by hydroxy, or O(C₁₋₄)alkyl wherein alkyl is unsubstituted or substituted by halogen,
   Y is -CH₂-, -C(O)-, -CH(OH)-, -C(=NOH)-, O or S,
   R₄ is (C₄₋₁₄)alkyl or (C₄₋₁₄)alkenyl;
   or a pharmaceutically acceptable salt or hydrate thereof;
Compounds as disclosed in WO 02/06268 or JP-14316985, e.g. a compound of formula VII 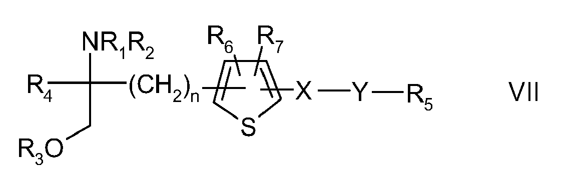 wherein
   each of R₁ and R₂, independently, is H or an amino-protecting group;
   R₃ is hydrogen or a hydroxy-protecting group;
   R₄ is (C₁₋₆)alkyl;
   n is an integer of 1-6;
   X is ethylene, vinylene, ethynylene, a group having a formula - D-CH₂- (wherein, D is carbonyl, a group having a formula -CH(OH)-, O, S or N; aryl or aryl substituted by three members selected from group a as defined hereinafter;
   Y is single bond, C₁₋₁₀alkylene, C₁₋₁₀alkylene which is substituted by one to three substituents selected from groups a and b, C₁₋₁₀alkylene having O or S in the middle or end of the carbon chain, or C₁₋₁₀alkylene having O or S in the middle or end of the carbon chain which is substituted by one to three substituents selected from groups a and b;
   R₅ is hydrogen, cycloalkyl, aryl, heterocycle, cycloalkyl substituted by one to three members selected from groups a and b, aryl substituted by one to three members selected from groups a and b, or heterocycle substituted by one to three members selected from groups a and b; and
   each of R₆ and R₇, independently, is H or a substituent selected from group a;
   <group a> is halogen, lower alkyl, halogeno lower alkyl, lower alkoxy, lower alkylthio, carboxyl, lower alkoxycarbonyl, hydroxy, lower aliphatic acyl, amino, mono-lower alkylamino, di-lower alkylamino, lower aliphatic acylamino, cyano and nitro;
   <group b> is cycloalkyl, aryl, heterocycle, each being optionally substituted by up to three substituents selected from group a;
   with the proviso that when R₅ is hydrogen, Y is either a single bond or linear C₁₋₁₀ alkylene,
   e.g. (2R)-2-amino-4-[3-(4-cyclohexyloxybutyl)benzo[b]thien-6-yl]-2-methylbutan-1-ol,
   or a pharmacologically acceptable salt or ester thereof.

When in the compounds of formula I the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy. Acyl may be a residue R-CO-, wherein R is C₁₋₆alkyl, C₃₋₆cycloalkyl, phenyl or phenyl-C₁₋₄alkyl.

Preferred compounds of formula I are those wherein R₁ is a straight or branched, preferably straight, chain alkyl having 13-20 carbon atoms, optionally substituted by nitro, halogen, amino, hydroxy or carboxy, and, more preferably those wherein R₁ is phenylalkyl substituted by a straight or branched C₆₋₁₄alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

A preferred compound of formula I is 2-amino-2-tetradecyl-1,3-propanediol. A particularly preferred S1 P receptor agonist of formula I is 2-amino-2-[2-(4-octylphenylethyl)]propane-1,3-diol in free form or in a pharmaceutically acceptable salt form (referred to hereinafter as Compound A), e.g. the hydrochloride, i.e. FTY720, as shown:

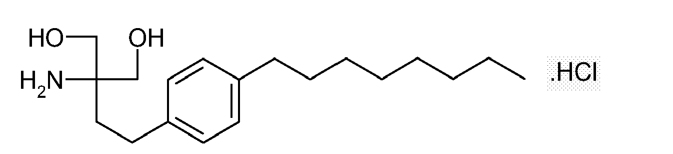

A preferred compound of formula II is one wherein each of R₂ to R₅ is H and m is 4, i.e. 2-amino-2-{2-[4-(1-oxo-5-phenylpentyl)phenyl]ethyl}propane-1,3-diol (referred to hereinafter as Compound B), in free form or in a pharmaceutically acceptable salt form, e.g. the hydrochloride.

A preferred compound of formula IVa is the Compound A-phosphate (R₂ is H, R₃ is OH, X is O, R₁ₐ and R_{1b} are OH). A preferred compound of formula V is Compound B-phosphate (R₁ is CH₂OH, R₃ is H, X is O, m is 1, R₂ is phosphate and R is 2-[4-(1-oxo-5-phenylpentyl)phenyl]ethyl).

When the compounds of formulae I to VII have one or more asymmetric centers in the molecule, the various optical isomers, as well as racemates, diastereoisomers and mixtures thereof are embraced.

Examples of pharmaceutically acceptable salts of the compounds of formulae I to VII include salts with inorganic acids, such as hydrochloride, hydrobromide and sulfate, salts with organic acids, such as acetate, fumarate, maleate, benzoate, citrate, maleate, methanesulfonate and benzenesulfonate salts, or, when appropriate, salts with metals, such as sodium, potassium, calcium and aluminium, salts with amines, such as triethylamine and salts with dibasic amino acids, such as lysine. The compounds and salts of the present invention encompass hydrate and solvate forms.

The composition of the present invention may comprise one or more salts and/or free acid of the S1 P agonists and//or modulator.

The composition of the invention preferably contains 0.01 to 20% by weight of S1 P receptor agonists, more preferably 0.1 to 10%, e.g. 0.5 to 5% by weight, based on the total weight of the composition.

### Core tablet formulation comprising an S1P receptor agonist or modulator

An example of a core tablet comprising an S1 P receptor agonist or modulator formulation may be found in WO 2004089341, which describes the formulation of an S1P agonist or modulator with a sugar alcohol.

The sugar alcohol may act as a diluent, carrier, filler or bulking agent, and may suitably be mannitol, maltitol, inositol, xylitol or lactitol, preferably a substantially non-hygroscopic sugar alcohol, e.g. mannitol (D-mannitol). A single sugar alcohol may be used, or a mixture of two or more sugar alcohols, e.g a mixture of mannitol and xylitol, e.g. in a ratio of 1:1 to 4:1.

In one embodiment, where the core tablet composition is in unit dosage form, each unit dosage will suitably contain 0.5 to 10 mg of the S1 P receptor agonist.

Possible manufacturing of the tablet cores comprises blending of all ingredients and further compressing to tablets, and granulation and further compressing of the granules to tablets.

Preferably, the composition is stable for at least six months at ambient temperature.

In one embodiment of the present invention, the solid formulation may be formulated to have a fast disintegration rate.

Preferably, the active ingredient dose ranges from 0 to 1000 mg.

The compositions of the invention may show good stability characteristics as indicated by standard stability trials, for example having a shelf life stability of up to one, two or three years, and even longer. Stability characteristics may be determined, e.g. by measuring decomposition products by HPLC analysis after storage for particular times, at particular temperatures, e.g. 20°, 40° or 60°C.

In one aspect, the present invention relates to a process for producing a coated pharmaceutical composition, comprising:
(a) mixing an S1 P receptor agonist with a sugar alcohol;
(b) milling and/or granulating the mixture obtained in (a); and
(c) mixing the milled mixture obtained in (b) with a lubricant
(d) optionally, another solvent, a flavor or a preservative, in a propylene glycol and addition of glycerin; and
(e) applying a coating composition of the present invention.

By using this process, a preparation having a good level of content and blend uniformity (i.e. for example, a substantially uniform distribution of the S1 P receptor agonist throughout the composition), dissolution time and stability is obtained.

In the case of a tablet core composition comprising the S1 P receptor agonist, e.g. 2-amino-2-[2-(4-octylphenyl)ethyl]propane-1,3-diol, hydrochloride, may optionally be micronized, and/or pre-screened, e.g. with a 400 to 500 µm mesh screen, before step (a) in order to remove lumps. The mixing step (a) may suitably comprise blending the S1 P receptor agonist and the sugar alcohol, e.g. mannitol in any suitable blender or mixer for e.g. 100 to 400 revolutions.

The process may be carried out by dry mixing the components. In this embodiment the milling step (b) may suitably comprise passing the mixture obtained in (a) through a screen, which preferably has a mesh size of 400 to 500 µm. Process step (a) may comprise the step of mixing the total amount of S1 P receptor agonist at first with a low amount of sugar alcohol, e.g. from 5 to 25% by weight of the total weight of sugar alcohol, in order to form a pre-mix. Subsequently the remaining amount of sugar alcohol is added to the pre-mix. Step (a) may also comprise the step of adding a binder solution, e.g. methylcellulose and/or xylitol, e.g. an aqueous solution, to the mixture.

The milled mixture obtained in (b) may optionally be blended once more before mixing with the lubricant. The lubricant, e.g. magnesium stearate, is preferably pre-screened, e.g. with a 800 to 900 µm screen, before mixing.

Alternatively, a wet granulation process is employed. In this embodiment, the S1 P receptor agonist is preferably first dry-mixed with the desired sugar alcohol, e.g. mannitol, and the obtained sugar alcohol/S1P receptor agonist mixture is then dry-mixed with a binder such as hydroxypropyl cellulose or hydroxypropylmethyl cellulose. Water is then added and the mixture granulated, e.g. using an automated granulator. The granulation is then dried and milled.

If desirable, an additional amount of binder may be added in step (c) to the mixture obtained in (b).

The process may comprise a further step of tabletting or encapsulating the mixture obtained in (c), e.g. into a hard gelatin capsule using an automated encapsulation device. The capsules may be coloured or marked so as to impart an individual appearance and to make them instantly recognizable. The use of dyes can serve to enhance the appearance as well as to identify the capsules. Dyes suitable for use in pharmacy typically include carotinoids, iron oxides, and chlorophyll. Preferably, the capsules of the invention are marked using a code.

The pharmaceutical compositions of the present invention are useful, either alone or in combination with other active agents, for the treatment and prevention of conditions e.g. as disclosed in US 5,604,229, WO 97/24112, WO 01/01978, US 6,004,565, US 6,274,629 and JP-14316985, the contents of which are incorporated herein by reference.

### Use of the composition

The compositions of the present invention may be used for aiding the oral administration of formulations comprising a sphingosine-1 phosphate receptor agonist or modulator.

In one embodiment of the present invention, the oral composition described herein promotes the absorption and distribution of the S1 P agonist and/or modulator through the blood brain barrier and into the brain.

In particular, the pharmaceutical compositions are useful for:
a) treatment and prevention of organ or tissue transplant rejection, for example for the treatment of the recipients of heart, lung, combined heart-lung, liver, kidney, pancreatic, skin or corneal transplants, and the prevention of graft-versus-host disease, such as sometimes occurs following bone marrow transplantation; particularly in the treatment of acute or chronic allo- and xenograft rejection or in the transplantation of insulin producing cells, e.g. pancreatic islet cells;
b) treatment and prevention of autoimmune disease or of inflammatory conditions, e.g. multiple sclerosis, arthritis (for example rheumatoid arthritis), inflammatory bowel disease, hepatitis, etc.;
c) treatment and prevention of viral myocarditis and viral diseases caused by viral mycocarditis, including hepatitis and AIDS.

The invention is, in one embodiment, related to the treatment of inflammatory conditions. In one example, the invention is related to compositions for the control and/or suppression of mast cell activation and secretion for the relief of inflammatory conditions, e.g., in the brain as in multiple sclerosis.

There is also provided a method of protecting multiple sclerosis subjects against neurodegerative brain inflammation, comprising the administration to said subjects a composition as described herein, for example a composition comprising an S1P agonist and/or modulator.

The composition of the present invention and any concentrate for dilution and pharmaceutical solution made therefrom, may be administered in an amount which is therapeutically effective against a disease or condition which can be treated by administration of the S1 P receptor agonist.

The exact amount of S1P receptor agonist or pharmaceutically acceptable salt thereof to administer can vary widely. The dose may depend on the particular compound, route of administration, the rate of administration, the strength of the particular concentrate or pharmaceutical solution employed, the nature of the disease or condition being treated, and the sex, age and body weight of the patient. The dose may also depend on the existence, nature and extent of any adverse side-effects that may accompany the administration of the concentrate or pharmaceutical formulation. Typically, a dose of 0.5 to 5 mg of S1 P receptor agonist, e.g. Compound A, are administered to children.

The composition of the present invention and any concentrate for dilution and respective pharmaceutical solution may be used in combination with other immunosuppressant(s), steroid(s) such as prednisolone, methylprednisolone, dexamethasone, hydrocortisone and the like, or nonsteroidal anti-inflammatory agent. The administration of a combination of active agents may be simultaneous or consecutive, with either one of the active agents being administered first. The dosage of the active agents of a combination treatment may depend on effectiveness and site of action of each active agent, as well as synergistic effects between the agents used for combination therapy.

The invention will now be described with reference to the following specific embodiments.

### Example 1

Micronized Compound 2-amino-2-[2-(4-octylphenyl)ethyl]propane-1,3-diol, hydrochloride salt (FTY720), is screened mixed with the microcrystalline cellulose agent, e.g. Avicel PH 102. The mixture is then milled in a Frewitt MGI device (Key International Inc. USA) using a 30 mesh screen. Magnesium stearate is screened using a 20 mesh screen and blended with the FTY720/cellulosemixture. Crosscarmellose is the blended to produce a product composition.

An example for a 6 mm round, 80 mg tablet core obtained by direct compression is shown below:

| Ingredient | mg/dose |
|---|---|
| FTY720 HCl | 1.40 |
| Microcrystalline cellulose, e.g. Avicel PH 102 | 73.80 |
| Magnesium stearate | 0.80 |
| Crosscarmellose | 4.00 |

As an alternative, a core tablet composition may be compacted on a tablet press using a 7 mm die to form 120 mg tablets, an example of which may be:

| Ingredient | mg/dose |
|---|---|
| FTY720 HCl | 1.40 |
| Mannitol M200 | 116.20 |
| Magnesium stearate | 2.40 |

1 mg of FTY720 in free form is equivalent to 1.12 mg of FTY720 HCl salt.

### Example 2

In a further example, the process of example 1 is repeated except that the magnesium stearate is replaced by Cutina® (hydrogenated castor oil).

### Example 3

In a further Example, the tablets are prepared as described in examples 1 and 2, except that FTY720 is replaced in each case by 2-amino-2-{2-[4-(1-oxo-5-phenylpentyl)phenyl]ethyl}propane-1,3-diol hydrochloride.

### Examples 4 to 7

Tablets containing the following ingredients (in mg) are produced:

| | Example 4 | Example 5 | Example 6 | Example 7 |
|---|---|---|---|---|
| FTY720 | 1 | 1 | 1 | 1 |
| D-mannitol | 62.3 | 62.3 | 62.0 | 62.0 |
| Xylitol* | 26.7(5.4) | 26.7(5.4) | 26.6 | 26.6 |
| Methylcellulose | - | - | 0.4 | 0.4 |
| Microcrystalline cellulose | 24.0 | - | 24.0 | - |
| Low-substituted Hydroxypropylcellulose | - | 24.0 | - | 24.0 |
| Hydrogenated oil | 6.0 | 6.0 | 6.0 | 6.0 |
| Total | 120.0 | 120.0 | 120.0 | 120.0 |

| | | | | |
|---|---|---|---|---|
| * The amount of xylitol indicated in brackets was used as a binder. | | | | |

FTY720, D-mannitol and xylitol are placed in a fluid-bed granulator (MP-01 model, Powrex), mixed for five minutes, and granulated under spray of binder solution, followed by drying till the exhaust temperature reaches 40°C. The granulation conditions are as shown below. Dried powder is passed through a 24-mesh sieve, added to the specified amount of filler and lubricant, and mixed in a mixer (Tubular Mixer, WAB) for three minutes to make the powder for compression.

The resulting powder is compressed by a tabletting machine (Cleanpress correct 12 HUK, Kikushui Seisakusho) with a punch of 7 mm i.d. x 7.5 mm R at a compression force of 9800 N.

### Granulation conditions:

| Item | Setting |
|---|---|
| Charge-in amount | 1170 g |
| Volume of intake-air | 50 m³/min |
| Temperature of intake-air | 75°C |
| Flow rate of spray solution | 15 mL/min |
| Spray air pressure | 15 N/cm² |
| Spray air volume | 30 L/min |
| Volume of binder solution | 351 mL |

### Example 8

An example powder coating composition:
The components are premixed under high shear, then wet granulated by mixing under high shear with water. The granulated mixture is dried in fluid bed drier to reduce the moisture content to below 3% by weight. The dried granules are milled and micronised to a powder.

| Ingredient | Composition (% w/w) |
|---|---|
| Ammonio-methacrylate co-polymer, e.g. Eudragit RS | 46.5 |
| hydroxy propyl cellulose, e.g. Klucel | 28.0 |
| titanium dioxide | 15.0 |
| aluminium lake | 5.0 |
| polyethylene glycol 6000 | 5.0 |
| colloidal silicon dioxide, e.g. Aerosil 200 | 0.5 |

### Example 9:

An example powder coating composition:

| Ingredient | Composition (% w/w) |
|---|---|
| Ammonio-methacrylate co-polymer, e.g. Eudragit RS | 39.75 |
| hydroxy propyl cellulose, e.g. Klucel | 39.75 |
| titanium dioxide | 15.0 |
| aluminium lake | 5.0 |
| colloidal silicon dioxide, e.g. Aerosil | 0.5 |

### Example 10:

An example liquid coating composition (aqueous dispersion):
At the fusing or drying stations, energy is imparted to the core surfaces to fuse the powder or dry the liquid and provide a uniform coating on the exposed surfaces of the core. The energy is provided by focused radiation preferably in the infra-red region; the energy power requirement will be determined largely by the coating material. After fusing or drying, the coating is set by cooling, using an air blower.

| Ingredient | Composition (% w/w) |
|---|---|
| hydroxypropylmethylcellulose | 70 |
| glycerol | 7 |
| iron oxide yellow | 23 |

## Claims

1. An oral pharmaceutical composition comprising an S1P agonist and/or modulator, wherein the composition comprises a coating comprising:
(a) one or more polymer resins
(b) one or more metal oxides.

2. The oral pharmaceutical composition of claim 1, wherein the one or more polymer resins comprises a methycrylic acid co-polymer.

3. The oral pharmaceutical composition of claim 1 or 2, wherein the one or more polymer resins comprises a cellulose.

4. The oral pharmaceutical composition of any preceding claim, which further comprises;
(d) a polyethylene glycol or a sugar alcohol

5. The oral pharmaceutical composition of any preceding claim, which further comprises;
(e) a colloidal silicon dioxide.

6. The oral pharmaceutical composition of any preceding claim, wherein the ammonio-methacrylate co-polymer is present in an amount of from 35 to 50 %w/w.

7. The oral pharmaceutical composition of any preceding claim, wherein the hydroxyl propyl cellulose is present in an amount of from 25 to 45 %w/w.

8. The oral pharmaceutical composition of any preceding claim, wherein the one or more metal oxides is present in an amount of from 15 to 25 %w/w.

9. The oral pharmaceutical composition of any preceding claim titanium dioxide is present in an amount of from 12.5 to 17.5 %w/w.

10. The oral pharmaceutical composition of any preceding claim, wherein aluminium lake is present in an amount of from 2.5 to 7.5 %w/w.

11. The oral pharmaceutical according to any preceding claim, wherein the cellulose is selected from hydroxypropyl cellulose, hydroxypropylmethyl cellulose or methyl cellulose.

12. The oral pharmaceutical composition of any preceding claim, wherein the polyethylene glycol, if present, is present in an amount of from 0.01 to 10 %w/w.

13. The oral pharmaceutical composition of any preceding claim, wherein the colloidal silicon dioxide, if present, is present in an amount of from 0.01 to 1 %w/w.

14. The oral pharmaceutical of any preceding claim, wherein the coating comprises:
| Ingredient | Composition (% w/w) |
|---|---|
| Ammonio-methacrylate co-polymer, e.g. Eudragit RS | 46.5 |
| hydroxy propyl cellulose, e.g. Klucel | 28.0 |
| titanium dioxide | 15.0 |
| aluminium lake | 5.0 |
| polyethylene glycol 6000 | 5.0 |
| colloidal silicon dioxide, e.g. Aerosil 200 | 0.5 |

15. The oral pharmaceutical of any one of claims 1 to 13, wherein the coating comprises:
| Ingredient | Composition (% w/w) |
|---|---|
| Ammonio-methacrylate co-polymer, e.g. Eudragit RS | 39.75 |
| hydroxy propyl cellulose, e.g. Klucel | 39.75 |
| titanium dioxide | 15.0 |
| aluminium lake | 5.0 |
| colloidal silicon dioxide, e.g. Aerosil | 0.5 |

16. An oral pharmaceutical composition comprising an S1P agonist and/or modulator, wherein the composition comprises a liquid coating comprising:
(a) hydroxypropylmethylcellulose
(b) glycerol
(c) a metal oxide

17. The oral pharmaceutical composition of claim 16, wherein the hydroxypropylmethylcellulose is present in an amount of from 60 to 80 %w/w.

18. The oral pharmaceutical composition of claim 16 or 17, wherein the glycerol is present in an amount of from 4 to 10 %w/w.

19. The oral pharmaceutical composition of claim 16, 17 or 18, wherein the metal oxide is present in an amount of from 15 to 30 %w/w.

20. The oral pharmaceutical composition of any one of claims 16 to 19, wherein the metal oxide is iron oxide yellow.

21. The oral pharmaceutical composition of any one of claims 16 to 20, wherein the coating comprises:
| Ingredient | Composition (% w/w) |
|---|---|
| hydroxypropylmethylcellulose | 70 |
| glycerol | 7 |
| iron oxide yellow | 23 |

22. The oral pharmaceutical according to any preceding claim , wherein the S1P receptor agonist comprises 2-amino-2-[2-(4-octylphenyl)ethyl]propane-1,3-diol or 2-amino-2-{2-[4-(1-oxo-5-phenylpentyl)-phenyl]ethyllpropane-1,3-diol or a pharmaceutically acceptable salt thereof.

23. The oral pharmaceutical composition according to claim 21 or 22, comprising 0.5 to 5% by weight of the S1 P receptor agonist.

24. A oral pharmaceutical composition according to any of claim 20 to 23, in the form of a tablet.

25. A method of treating a subject in need of immunosuppression, comprising administering to the subject a composition according to any one of claims 21 to 24.

26. Use of a pharmaceutical composition according to any one of claims 21 to 24 for the preparation of a medicament for the prevention or treatment of organ or tissue transplant rejection, or for the prevention or treatment of an inflammatory or autoimmune disease.

27. A process for producing a coated pharmaceutical tablet for oral administration, comprising:
(a) preparing a core tablet comprising an S1 P agonist and/or modulator
(b) applying a coating as described in any of claims 1 to 24.

28. A method of protecting multiple sclerosis subjects against neurodegerative brain inflammation, comprising the administration to said subjects the composition of any of claims 1 to 24.
